# EUROPEAN PATENT APPLICATION

(11) **EP 1 780 289 A2**
(43) Date of publication of application: **02.05.2007**
(21) Application number: 06022464.9
(22) Date of filing: 27.10.2006
(51) Int. Cl.: C12Q 1/68

(54) **Method for assaying Reg IV mRNA**

(30) Priority: 28.10.2005 JP 2005314343
(71) Applicant: TOSOH CORPORATION, Shunan-shi Yamaguchi-ken 746-8501 (JP)
(72) Inventor: Une, Kurando, Atsugi-shi Kanagawa (JP); Saito, Juichi, Yamato-shi Kanagawa (JP); Hayashi, Toshinori, Sagamihara-shi Kanagawa (JP)
(74) Representative: Pautex Schneider, Nicole Véronique

(57) **Abstract**

In an RNA amplification process comprising steps of using a first primer and a second primer, at least one of which has a promoter sequence at the 5' end, and reverse transcriptase, to produce double-stranded DNA containing the promoter sequence, using the double-stranded DNA as template to produce an RNA transcript with RNA polymerase, and using the RNA transcript in turn as template for DNA synthesis with the reverse transcriptase to produce the double-stranded DNA, the amount of amplified RNA product is measured with an intercalating fluorescent dye-labeled nucleic acid probe.

## Description

### Technical Field

The present invention relates to a method for assaying Regenerating islet-derived family, member 4 (regenerating gene type 4) mRNA (Reg IV mRNA) in a convenient, isothermal, single-stage and rapid manner. The invention pertains to the field of medicine and particularly to the field of clinical diagnosis or cancer research, and is useful for detection of cancer cells.

### Background Art

Regenerating islet-derived family, member 4 (regenerating gene type 4) (hereinafter, "Reg IV") is a member of the Reg gene family, and it is a secreted protein belonging to the C-type lectin superfamily. The Reg gene family proteins function as lectins, apoptosis resistance factors and growth factors in pancreatic β cells, neurons, gastrointestinal epithelial cells and the like. Expression of Reg IV has been found in gastrointestinal tissue, and its expression level is reported to be augmented in gastric cancer, colon cancer and other cancers (see Zang YW. et al, (2003) World J. Gastroenterol., 9, 2635-2641).

In recent years, measurement of Reg IV mRNA levels using RT-PCR has revealed increased Reg IV mRNA levels in gastrointestinal cancers such as gastric cancer and colon cancer (see Oue N. et al, (2004) Cancer Res., 64, 2397-2405 and Violette S. et al, (2002) Int. J. Cancer, 103, 185-193). In other words, these discoveries suggest that assay of Reg IV mRNA is effective as a marker for gastric cancer and colon cancer.

One means for highly sensitive assay of Reg IV mRNA is a method of amplifying Reg IV mRNA by RT-PCR and measuring the amount of amplification product, but this generally requires a two-stage process including a reverse transcription (RT) stage and a PCR stage, which not only complicates the procedure and lowers reproducibility, but also raises the risk of secondary contamination. The RT and PCR stages combined take 2 hours or longer in most cases, and the method is therefore unsuitable for processing of multiple specimens or reducing the cost of examinations.

Assay of target RNA is commonly accomplished by Real-time RT-PCR whereby the PCR stage is carried out in the presence of an intercalating fluorescent dye and increase in fluorescence is measured, but this problem is associated with detection of non-specific amplification products such as primer dimers. Also, because DNA is also amplified in RT-PCR, the chromosomal DNA must be completely removed by DNase treatment or the like during the nucleic acid extraction step if the goal is to amplify only mRNA, and this has led to a more complex procedure for nucleic acid extraction. In addition, PCR requires abrupt increase/decrease in the reaction temperature, which has constituted an obstacle against power savings and cost reduction for automated reactors.

The NASBA method (see Japanese Patent No. 2650159 and Japanese Patent No. 3152927) and TMA method (see Japanese Patent No. 3241717) have been reported as methods for amplifying only RNA in an isothermal manner. These RNA amplification methods employ a chain reaction wherein a primer including the promoter sequence for the target RNA, reverse transcriptase and if necessary Ribonuclease H (RNaseH) are used for synthesis of double-stranded DNA containing the promoter sequence, RNA polymerase is used for synthesis of RNA containing the specified nucleotide sequence of the target RNA, and the RNA is in turn used as template for synthesis of double-stranded DNA containing the promoter sequence. After RNA amplification, the amplified RNA is detected by electrophoresis or a hybridization method using a nucleic acid probe bound to a detectable label.

These RNA amplification methods are suitable for convenient mRNA assay since they amplify only RNA in an isothermal, single-stage manner, but detection by hybridization methods and the like require complex procedures and do not allow highly reproducible quantitation.

A convenient method for amplification and assay of mRNA is the method developed by Ishiguro et al. (see Japanese Unexamined Patent Publication No. 2000-14400 and Ishiguro T. et al, (2003) Anal. Biochem., 314, 77-86). In this method, RNA amplification is carried out in the presence of a nucleic acid probe which is labeled with an intercalating fluorescent dye and is designed so that when it forms a complementary double strand with the target nucleic acid, the intercalating fluorescent dye portion undergoes a change in fluorescent property by intercalating into the complementary double strand, and the change in fluorescent property is measured, whereby it is possible to simultaneously accomplish RNA amplification and assay in a convenient, isothermal and single-stage manner in a sealed vessel.

### Disclosure of the Invention

Although the aforementioned Reg IV mRNA assays are useful for diagnosis of gastrointestinal cancers such as gastric cancer and colon cancer, the use of RT-PCR requires a two-stage process, a complex procedure and abrupt increase/decrease in reaction temperature, and these conditions not only entail the risk of secondary contamination and have poor reproducibility, but are also obstacles to development of more convenient measurement and automation. It is an object of the present invention to overcome the aforementioned problems by providing a method for assaying Reg IV mRNA in a convenient, rapid, isothermal and single-stage manner.

As a result of much diligent research directed toward solving the aforementioned problems, the present inventors have constructed a method for assaying Reg IV mRNA in a convenient, rapid, isothermal and single-stage manner which applies the RNA amplification method explained above. Specifically, by measuring the level of amplified RNA product obtained by an RNA amplification process wherein a first primer and second primer (at least one of which has a promoter sequence at the 5' end) are used to produce double-stranded DNA containing the promoter sequence, the double-stranded DNA is used as template to produce an RNA transcript, and the RNA transcript in turn is used as template for DNA synthesis to produce the double-stranded DNA, it has become possible to assay Reg IV mRNA in a convenient, isothermal and single-stage manner.

According to the present invention it has become possible to achieve highly sensitive assay of Reg IV mRNA in an isothermal, single-stage, convenient and rapid manner. The invention can therefore be applied for diagnosis of gastric cancer, colon cancer and other cancers, and is thus useful as a cancer marker. Since the invention can be carried out in a single stage and in a sealed vessel, it is possible to minimize the risk of contamination of the environment by amplification product as a secondary contaminant. Furthermore, since the method is also carried out in a single-stage, convenient and rapid manner, multiple specimens can be processed and it is possible to minimize the number of procedures that can potentially reduce the reproducibility. In addition, since the RNA amplification method of the invention amplifies only RNA, it is possible to achieve stringent amplification and assay of mRNA without a step of completely removing double-stranded DNA as is required in RT-PCR. In other words, the method of the invention is optimal for highly sensitive and rapid expression analysis. Moreover, because it can be carried out in an isothermal and single-stage manner, there is no need to provide a thermal cycling mechanism as in PCR, and automation is thus facilitated.

### Brief Explanation of the Drawings

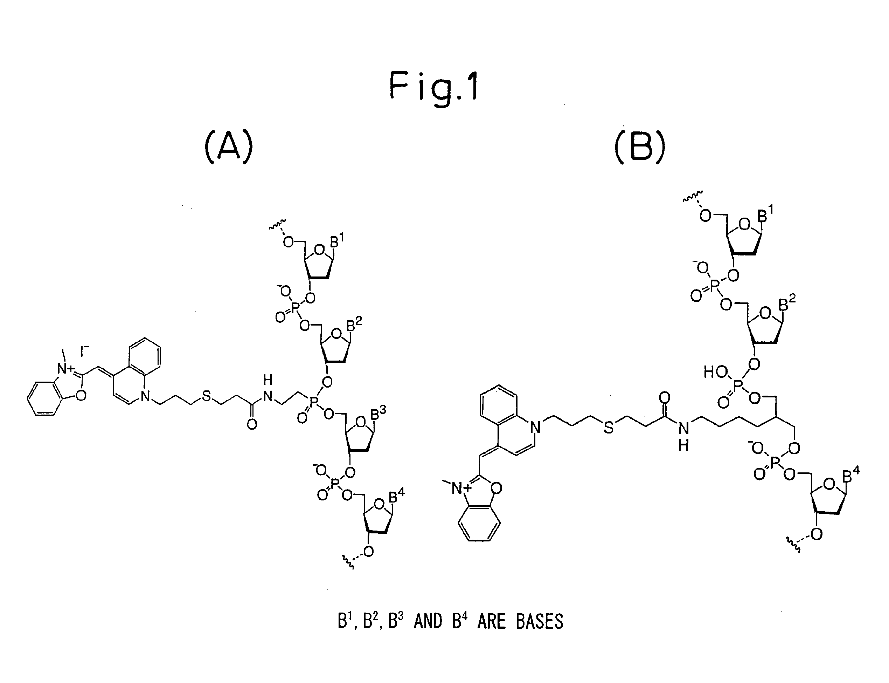

Fig. 1 shows the structure of the intercalating fluorescent dye-labeled nucleic acid probe prepared in Example 2. B1, B2, B3 and B4 represent bases. (A) Probe having intercalating fluorescent dye (oxazole yellow) bonded via a linker to a phosphate diester portion according to the method of Ishiguro et al. (Ishiguro T. et al, (1996) Nucleic Acids Res., 24, 4992-4997). In order to prevent 3'-terminal -OH group extension reaction, the 3'-terminal -OH group was modified with glycolic acid. (B) Probe having an amino group introduced using commercially available Label-ON Reagents (Clontech), and oxazole yellow bonded according to the method of Ishiguro et al. (Ishiguro T. et al, (1996) Nucleic Acids Res., 24, 4992-4997). Here, the amino group was introduced at the nucleoside-lacking portion at the site of introduction (B3 in the drawing). In order to prevent 3'-terminal -OH group extension reaction, the 3'-terminal -OH group was modified with biotin.

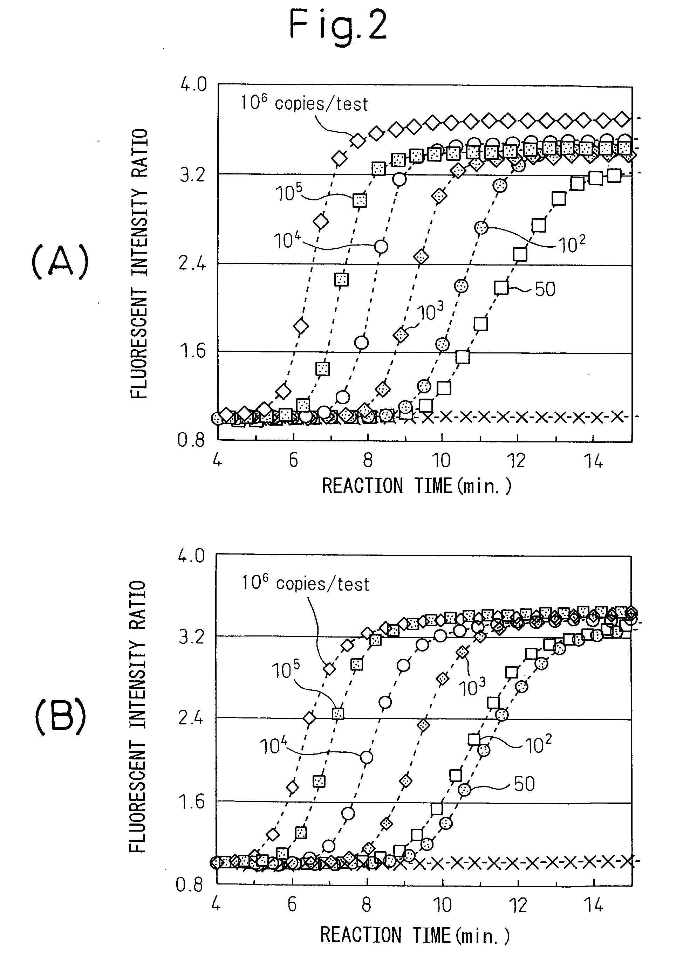

Fig. 2 shows fluorescence profiles obtained as a result of the measurement of Example 3. Results of carrying out RNA amplification according to the invention simultaneously with periodic measurement of fluorescent intensity (excitation wavelength: 470 nm, fluorescent wavelength: 520 nm). The horizontal axis represents reaction time, and the vertical axis represents fluorescent intensity ratio (fluorescent intensity of reaction mixture/background fluorescence). The numbers of copies in the graphs represent the initial numbers of copies of Reg IV RNA (including base numbers 1-1275) used (calculated by absorbance at 260 nm). Fig. 2A shows the results using reaction mixture composition (A), and Fig. 2B shows the results using reaction mixture composition (B).

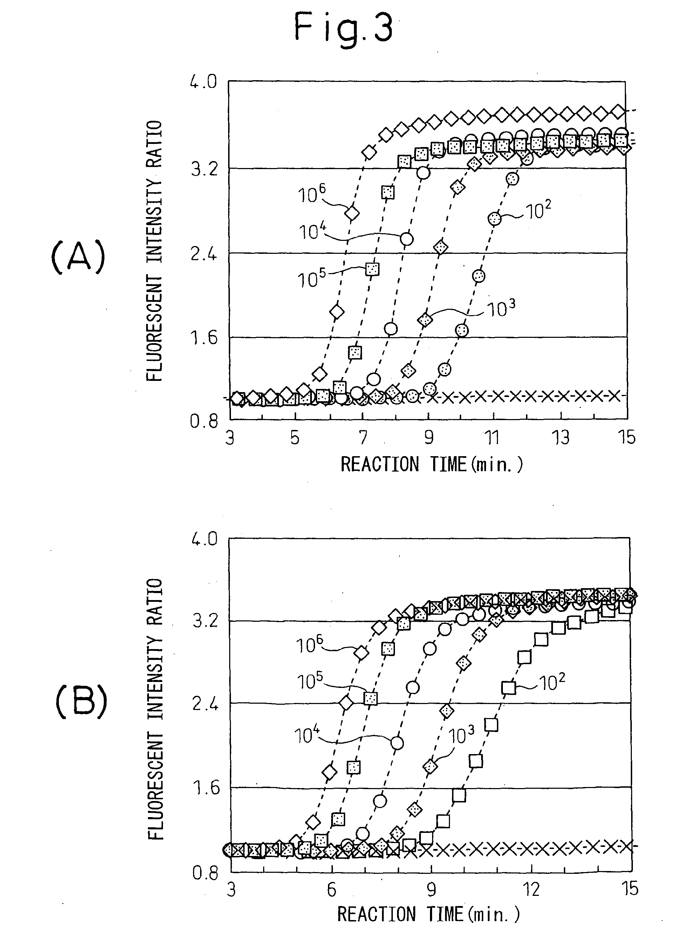

Fig. 3 shows fluorescence profiles obtained as a result of the measurement of Example 4. Results of carrying out RNA amplification according to the invention simultaneously with periodic measurement of fluorescent intensity (excitation wavelength: 470 nm, fluorescent wavelength: 520 nm). The horizontal axis represents reaction time, and the vertical axis represents fluorescent intensity ratio (fluorescent intensity of reaction mixture/background fluorescence). The numbers for the cases in the graphs represent the initial numbers of copies of Reg IV RNA (including base numbers 1-1275) used (calculated by absorbance at 260 nm). Fig. 3A shows the results using reaction mixture composition (A), and Fig. 3B shows the results using reaction mixture composition (B).

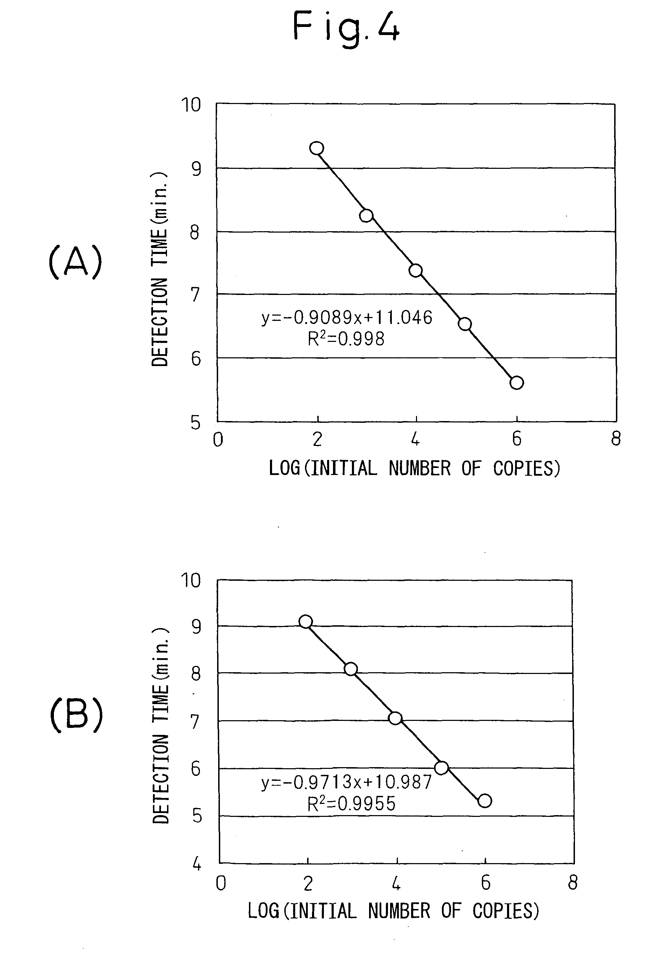

Fig. 4 is a pair of calibration curves obtained from the results of Fig. 3. With the detection time defined as time at which the fluorescent intensity ratio reached 1.2 in the results of Fig. 3, the detection time was plotted with respect to the logarithm of initial number of copies of standard RNA. The formulas in the graphs represent the primary regression equations and correlation coefficients. The initial number of copies of unknown sample is calculated from this calibration curve and the detection time of the unknown sample obtained by the present method. Fig. 4A shows the results using reaction mixture composition (A), and Fig. 4B shows the results using reaction mixture composition (B).

### Best Mode for Carrying Out the Invention

The present invention will now be explained in detail.

The invention is a method for assaying Reg IV mRNA present in a sample, the method comprising a step of using a first primer homologous to at least a portion downstream from the 5' end of a specified nucleotide sequence of the RNA and a second primer complementary to at least a portion upstream from the 3' end of the specified nucleotide sequence (where at least one of the first and second primers has a promoter sequence at the 5' end) to produce double-stranded DNA containing the promoter sequence and the specified nucleotide sequence downstream from the promoter sequence, a step of using the double-stranded DNA as template to produce an RNA transcript, a step of using the RNA transcript in turn as template for DNA synthesis to produce the double-stranded DNA, a step of nucleic acid amplification in which the aforementioned steps are repeated under conditions that simultaneously promote each of the steps, and a step of assaying the amount of the RNA transcript.

The sample according to the invention consists of nucleic acid extracted by a known method from a specimen such as blood, serum, plasma, tissue or lavage suspected of containing cancer cells.

The specified nucleotide sequence according to the invention consists of at least a partial sequence of Reg IV mRNA or the sequence complementary thereto, and it is the sequence of the region defined between the first primer and second primer. According to the invention, the RNA transcript from the specified nucleotide sequence is amplified. When the promoter sequence is added to the first primer, the Reg IV mRNA is preferably cleaved at the 5' end of the specific nucleic acid sequence before serving as the template for cDNA synthesis. The cleavage method is not particularly restricted, but it is preferably a method using an enzyme with Ribonuclease H (RNaseH) activity to cleave the RNA portion of an RNA-DNA hybrid formed by adding an oligonucleotide (a cleavage oligonucleotide) having a sequence complementary to the region overlapping and adjacent to the 5' end of the specified nucleotide sequence of Reg IV mRNA, and preferably the cleavage oligonucleotide used has its 3'-terminal -OH appropriately modified, for example, aminated, to prevent extension reaction.

The target nucleic acid according to the invention has a region in the specific base sequence that is not homologous or complementary to the first and second primers, while having a sequence that allows complementary binding with the intercalating fluorescent dye-labeled nucleic acid probe. Thus, the intercalating fluorescent dye-labeled nucleic acid probe is a sequence complementary to a portion of the specified nucleotide sequence according to the invention. According to one mode of the invention, therefore, when the specified nucleotide sequence is a sequence homologous to Reg IV mRNA, the intercalating fluorescent dye-labeled nucleic acid probe has a sequence containing at least 15 contiguous bases of the sequence listed as SEQ ID NO: 7, and when the specified nucleotide sequence is a sequence complementary to Reg IV mRNA, the intercalating fluorescent dye-labeled nucleic acid probe has a sequence containing at least 15 contiguous bases of a sequence complementary to the sequence listed as SEQ ID NO: 7.

At least one of the first and second primers according to the invention has a promoter sequence at the 5' end, and are oligonucleotides with sufficient complementarity to a sequence complementary to Reg IV mRNA in the case of the first primer and with sufficient complementarity to Reg IV mRNA in the case of the second primer. "Sufficient complementarity" means that complementary binding can occur with the specified nucleotide sequence or the sequence complementary to that sequence, under the reaction conditions (reaction temperature and composition of salts, etc.) for the nucleic acid amplification step of the invention. The reaction conditions may be, for example, hybridization conditions at 43°C in the presence of 60 mM Tris, 18 mM magnesium chloride, 100 mM potassium chloride, 1 mM DTT, as indicated in the examples described below.

In order to provide sufficient complementarity to a sequence complementary to Reg IV mRNA for the first primer, to Reg IV mRNA for the second primer and to the target nucleic acid for the intercalating fluorescent dye-labeled nucleic acid probe, the first primer preferably includes at least 15 contiguous bases of the sequence listed as SEQ ID NO: 1, 3 or 5, the second primer preferably includes at least 15 contiguous bases of the sequence listed as SEQ ID NO: 2, 4 or 6, and the intercalating fluorescent dye-labeled nucleic acid probe preferably includes at least 15 contiguous bases of the sequence listed as SEQ ID NO: 7, 8 or 9 or the sequence complementary to the sequence.

Preferably, there is employed a combination of sequences in which the first primer includes at least 15 contiguous bases of the sequence listed as SEQ ID NO: 1, the second primer includes at least 15 bases of the sequence listed as SEQ ID NO: 2 and the intercalating fluorescent dye-labeled nucleic acid probe includes at least 15 bases of the sequence listed as SEQ ID NO: 7 or of the sequence complementary to the sequence, a combination of sequences in which the first primer includes at least 15 contiguous bases of the sequence listed as SEQ ID NO: 3, the second primer includes at least 15 bases of the sequence listed as SEQ ID NO: 4 and the intercalating fluorescent dye-labeled nucleic acid probe includes at least 15 bases of the sequence listed as SEQ ID NO: 8 or of the sequence complementary to the sequence, or a combination of sequences in which the first primer includes at least 15 contiguous bases of the sequence listed as SEQ ID NO: 5, the second primer includes at least 15 bases of the sequence listed as SEQ ID NO: 6 and the intercalating fluorescent dye-labeled nucleic acid probe includes at least 15 bases of the sequence listed as SEQ ID NO: 9 or of the sequence complementary to the sequence. These combinations are preferred modes because of the positional relationships of sequences that bind in a complementary manner to Reg IV mRNA, but other combinations also allow amplification and assay of Reg IV mRNA.

The first primer may be an oligonucleotide consisting of at least 15 contiguous bases of the sequence listed as SEQ ID NO: 1, 3 or 5 with a deletion, substitution or addition of one or more nucleotides which is capable of binding specifically to the sequence complementary to the specified sequence, or an oligonucleotide that hybridizes with an oligonucleotide consisting of at least 15 contiguous bases of the sequence listed as SEQ ID NO: 1, 3 or 5 under highly stringent conditions and is capable of binding specifically to the sequence complementary to the specified sequence, while the second primer may be an oligonucleotide consisting of at least 15 contiguous bases of the sequence listed as SEQ ID NO: 2, 4 or 6 with a deletion, substitution or addition of one or more nucleotides which is capable of binding specifically to the specified sequence, or an oligonucleotide that hybridizes with an oligonucleotide consisting of at least 15 contiguous bases of the sequence listed as SEQ ID NO: 2, 4 or 6 under highly stringent conditions and is capable of binding specifically to the specified sequence.

When the targent of detection is RNA that is complementary to RNA of Reg IV, the first primer and second primer may be the complementary primers with their 5' and 3' ends reversed.

Highly stringent conditions may be, for example, hybridization conditions at 43°C in the presence of 60 mM Tris, 18 mM magnesium chloride, 100 mM potassium chloride, 1 mM DTT, as indicated in the examples described below.

The promoter sequence according to the invention is a sequence to which RNA polymerase binds to initiate transcription, and specific sequences for different RNA polymerases are known. There are no particular restrictions on the RNA polymerase, but common ones such as T7 phage RNA polymerase, T3 phage RNA polymerase and SP6 phage RNA polymerase are preferred, and their corresponding promoter sequences may be used.

The method for assaying Reg IV mRNA according to the invention requires certain enzymes (an enzyme having RNA-dependent DNA polymerase activity for single-stranded RNA template) (reverse transcriptase), an enzyme having RNaseH activity, an enzyme having DNA-dependent DNA polymerase activity for single-stranded DNA template, and an enzyme having RNA polymerase activity). Any of these enzymes may be enzymes having different activities, or a plurality of enzymes having each activity may be used. For example, an enzyme having RNA polymerase activity may be added to a reverse transcriptase having RNA-dependent DNA polymerase activity for single-stranded RNA template, RNaseH activity and DNA-dependent DNA polymerase activity for single-stranded DNA template, or an additional enzyme with RNaseH activity may be further added as a supplement. Preferred as the reverse transcriptase are AMV reverse transcriptase, M-MLV reverse transcriptase and their derivatives, from the standpoint of flexible utility.

When the reverse transcription reaction is carried out in the presence of the first primer and second primer, the second primer binds to the specified base sequence of Reg IV mRNA and carries out cDNA synthesis with the enzyme having RNA-dependent DNA polymerase activity. The RNA portion of the obtained RNA-DNA hybrid is degraded by the enzyme having RNaseH activity, and dissociates so that the first primer may bind to the cDNA.

Next, double-stranded DNA derived from the specified base sequence and containing the promoter sequence at the 5' end is produced by the enzyme having DNA-dependent DNA polymerase activity. The double-stranded DNA contains the specified base sequence downstream from the promoter sequence, and an RNA transcript derived from the specified base sequence is produced by the enzyme having RNA polymerase activity. The RNA transcript serves as template for the double-stranded DNA synthesis by the first and second primers, so that a series of reactions occur in a chain reaction and result in amplification of the RNA transcript.

In order to promote the chain reaction, it is of course necessary to add at least buffer solution, magnesium salt, potassium salt, nucleoside triphosphates and ribonucleoside triphosphates as known elements essential for each of the enzymes. In addition, additives such as dimethylsulfoxide (DMSO), dithiothreitol (DTT), bovine serum albumin (BSA), sugars and the like may be added to control the reaction efficiency.

For example, when using AMV reverse transcriptase and T7 RNA polymerase, the reaction temperature is preferably set in a range of 35°C-65°C, and most preferably it is set in a range of 40°C-44°C. The RNA amplification step proceeds isothermally, and the reaction temperature may be set to any desired temperature at which the reverse transcriptase and RNA polymerase exhibit their activity.

The amount of amplified RNA transcript can be measured by a known nucleic acid assay method. As such assay methods, there may be used methods employing electrophoresis or liquid chromatography, or hybridization methods employing nucleic acid probes labeled with detectable labels. But these procedures involve multiple steps, and because the amplification product is removed out of the system for analysis, there is a high risk of escape of the amplification product into the environment as a cause of secondary contamination. To overcome these problems it is preferred to use a nucleic acid probe designed so that its fluorescent property changes upon complementary binding to the target nucleic acid. As a more preferred method, there may be mentioned a method wherein the nucleic acid amplification step is carried out in the presence of a nucleic acid probe which is labeled with an intercalating fluorescent dye and is designed so that when it forms a complementary double strand with the target nucleic acid, the intercalating fluorescent dye portion undergoes a change in fluorescent property by intercalating into the complementary double strand, and the change in fluorescent property is measured (see Japanese Unexamined Patent Publication No. 2000-14400 and Ishiguro T. et al, (2003) Anal. Biochem., 314, 77-86).

There are no particular restrictions on the intercalating fluorescent dye, and there may be used common dyes such as oxazole yellow, thiazole orange, ethidium bromide and their derivatives. The change in fluorescent property may be a change in fluorescent intensity. For example, it is known that in the case of oxazole yellow, intercalation into double-stranded DNA causes a notable increase in fluorescence at 510 nm (excitation wavelength of 490 nm). The intercalating fluorescent dye-labeled nucleic acid probe is an oligonucleotide with sufficient complementarity to the RNA transcript, and it has a structure with the intercalating fluorescent dye bonded to the end, to a phosphate diester portion or to a base portion via an appropriate linker, and with the 3'-terminal -OH group suitably modified to prevent extension from the 3'-terminal -OH (see Japanese Unexamined Patent Publication HEI No. 8-211050 and Ishiguro T. et al, (1996) Nucleic Acids Res., 24, 4992-4997).

Labeling of the oligonucleotide with the intercalating fluorescent dye may be accomplished by introducing a functional group into the oligonucleotide by a known method and bonding the intercalating fluorescent dye thereto (see Japanese Unexamined Patent Publication No. 2001-13147 and Ishiguro T. et al, (1996) Nucleic Acids Res., 24, 4992-4997). The method of introducing the functional group may employ the commonly used Label-ON Reagents (Clontech Laboratories, Inc.).

As one mode of the invention, there is provided a method of adding to the sample an amplification reagent containing at least a first primer containing the T7 promoter sequence at the 5' end (including 15 contiguous bases of the sequence listed as SEQ ID NO: 1), a second primer (including 15 contiguous bases of the sequence listed as SEQ ID NO: 2), an intercalating fluorescent dye-labeled nucleic acid probe (including 15 contiguous bases of the sequence listed as SEQ ID NO: 7), a cleavage oligonucleotide (including 15 contiguous bases of a sequence selected from among SEQ ID NOS: 49-58, and having a sequence complementary to the region overlapping and adjacent to the 5' end of the specified nucleotide sequence), AMV reverse transcriptase, T7 RNA polymerase, buffer solution, magnesium salt, potassium salt, nucleoside triphosphates, ribonucleoside triphosphates and dimethylsulfoxide (DMSO), and preferably adding to the sample an amplification reagent containing at least a first primer containing the T7 promoter sequence at the 5' end (sequence listed as SEQ ID NO: 10), a second primer (sequence listed as SEQ ID NO: 11), an intercalating fluorescent dye-labeled nucleic acid probe (sequence listed as SEQ ID NO: 42), a cleavage oligonucleotide (sequence listed as SEQ ID NO: 50), AMV reverse transcriptase, T7 RNA polymerase, buffer solution, magnesium salt, potassium salt, nucleoside triphosphates, ribonucleoside triphosphates and dimethylsulfoxide (DMSO), or an amplification reagent containing at least a first primer containing the T7 promoter sequence at the 5' end (sequence listed as SEQ ID NO: 18), a second primer (sequence listed as SEQ ID NO: 33), an intercalating fluorescent dye-labeled nucleic acid probe (sequence listed as SEQ ID NO: 42), a cleavage oligonucleotide (sequence listed as SEQ ID NO: 54), AMV reverse transcriptase, T7 RNA polymerase, buffer solution, magnesium salt, potassium salt, nucleoside triphosphates, ribonucleoside triphosphates and dimethylsulfoxide (DMSO), and performing reaction at a constant reaction temperature of 35-65°C (preferably 40-44°C) while periodically measuring the fluorescent intensity of the reaction mixture.

As a different mode, there is provided a method of adding to the sample an amplification reagent containing at least a first primer containing the T7 promoter sequence at the 5' end (including 15 contiguous bases of the sequence listed as SEQ ID NO: 3), a second primer (including 15 contiguous bases of the sequence listed as SEQ ID NO: 4), an intercalating fluorescent dye-labeled nucleic acid probe (including 15 contiguous bases of the sequence listed as SEQ ID NO: 8), a cleavage oligonucleotide (including 15 contiguous bases of the sequence listed as SEQ ID NO: 59, and having a sequence complementary to the region overlapping and adjacent to the 5' end of the specified nucleotide sequence), AMV reverse transcriptase, T7 RNA polymerase, buffer solution, magnesium salt, potassium salt, nucleoside triphosphates, ribonucleoside triphosphates and dimethylsulfoxide (DMSO), and performing reaction at a constant reaction temperature of 35-65°C (preferably 40-44°C) while periodically measuring the fluorescent intensity of the reaction mixture.

As a still different mode, there is provided a method of adding to the sample an amplification reagent containing at least a first primer containing the T7 promoter sequence at the 5' end (including 15 contiguous bases of the sequence listed as SEQ ID NO: 5), a second primer (including 15 contiguous bases of the sequence listed as SEQ ID NO: 6), an intercalating fluorescent dye-labeled nucleic acid probe (including 15 contiguous bases of the sequence listed as SEQ ID NO: 9), a cleavage oligonucleotide (including 15 contiguous bases of a sequence selected from SEQ ID NOS: 60-64 and having a sequence complementary to the region overlapping and adjacent to the 5' end of the specified nucleotide sequence), AMV reverse transcriptase, T7 RNA polymerase, buffer solution, magnesium salt, potassium salt, nucleoside triphosphates, ribonucleoside triphosphates and dimethylsulfoxide (DMSO), and performing reaction at a constant reaction temperature of 35-65°C (preferably 40-44°C) while periodically measuring the fluorescent intensity of the reaction mixture.

In all of the modes mentioned above, the fluorescent intensity results as an increase curve from the initial RNA amount, and therefore by drawing a calibration curve using standard RNA of known concentration, it is possible to quantify the initial amount of RNA in an unknown sample. Moreover, since the fluorescent intensity is periodically measured, the measurement may be concluded at any desired point at which a significant increase in fluorescence is detected, and measurement results from the nucleic acid amplification and assay can be obtained usually within an hour, or within 30 minutes with an optimal system.

It is especially notable that all of the test materials in the assay reagent can be included in the same vessel. That is, the simple procedure of dispensing prescribed amounts of test materials into a single vessel will allow automatic amplification and detection of Reg IV mRNA to be conducted thereafter. The vessel may be constructed of, for example, a partially transparent material to allow external measurement of the signal emitted by the fluorescent dye, and a vessel that can be sealed after dispensing the test materials is particularly preferred to prevent contamination.

The RNA amplification and assay method according to the modes described above can be carried out in a single-stage and isothermal manner, and it is therefore more convenient than RT-PCR and is suitable for automation. However, because the reaction is carried out at a relatively low constant temperature of 35-65°C without denaturation and annealing as in RT-PCR, it is prone to non-specific amplification products such as primer dimers and to effects of the higher order structure of the target RNA, and therefore a much more elaborate design is necessary to construct the assay system compared to RT-PCR, for which reason it had not been previously possible to assay Reg IV mRNA in a rapid, convenient, isothermal and single-stage manner using the aforementioned RNA amplification and assay method. According to the present invention, it possible for the first time to assay Reg IV mRNA with high specificity and high sensitivity in a rapid, convenient, isothermal and single-stage manner.

### Examples

The present invention will now be further explained by examples, with the understanding that the invention is not limited by the examples.

### Example 1

Reg IV RNA was prepared by using double-stranded DNA containing Reg IV cDNA (base numbers 1-1275, according to the base numbers assigned for National Center Biotechnology Information Accession No. NM032044) downstream from SP6 phage RNA polymerase/promoter as template for in vitro transcription, and then the double-stranded DNA was completely digested by DNaseI treatment and the RNA was purified. The RNA was quantitated by measuring the absorbance at 260 nm.

The following examples deal with this RNA as the object of measurement, but they are entirely applicable for assay of Reg IV mRNA as the object of measurement according to the invention.

### Example 2

Oligonucleotide probes labeled with an intercalating fluorescent dye were prepared. Amino groups were introduced at the positions of the 12th G from the 5' end of SEQ ID NO: 43, the 10th A from the 5' end of SEQ ID NO: 44, the 12th A from the 5' end of SEQ ID NO: 45, the 11th C from the 5' end of SEQ ID NO: 46, the 10th A from the 5' end of SEQ ID NO: 47 and the 14th C from the 5' end of SEQ ID NO: 48, using Label-ON Reagents (Clontech Laboratories, Inc.), and the 3' ends were modified with biotin. Oxazole yellow was bonded to the amino groups by the method described in Ishiguro T. et al, (1996) Nucleic Acids Res., 24, 4992-4997 (Fig. 1B). Also, an oxazole yellow-labeled nucleic acid probe was prepared having oxazole yellow bonded via a linker to the phosphate diester portion between the 9th C and 10th A from the 5' end of the sequence listed as SEQ ID NO: 42, by the method described in Ishiguro T. et al, (1996) Nucleic Acids Res., 24, 4992-4997 (Fig. 1A).

### Example 3

The method of the present invention was used to detect different original numbers of copies of Reg IV RNA.

(1) The aforementioned Reg IV RNA (including base numbers 1-1275) was diluted to 50, 10², 10³, 10⁴, 10⁵ and 10⁶ copies/5 µl using an RNA diluent (10 mM Tris-HCl (pH 8.0), 1 mM EDTA, 0.25 U/µl ribonuclease inhibitor, 5 mM DTT), for use as RNA samples. The RNA diluent was used as the negative standard (0 copies).

(2) After dispensing 20 µl of reaction mixture with the following composition into a 0.5 ml-volume PCR tube (GeneAmp Thin-Walled Reaction Tubes, Perkin-Elmer), 5 µl of RNA sample was added.

Reaction mixture composition (A): Concentrations are final concentrations (in 30 µl) after addition of enzyme solution.
60 mM Tris-HCl (pH 8.6)
18 mM magnesium chloride
100 mM potassium chloride
1 mM DTT
0.25 mM dATP, dCTP, dGTP, dTTP each
3 mM ATP, CTP, UTP, GTP each
3.6 mM ITP
0.2 µM first primer (SEQ ID NO: 10): The first primer included the T7 polymerase-promoter sequence (SEQ ID NO: 65) added at the 5' end of the indicated nucleotide sequence.
0.2 µM second primer (SEQ ID NO: 11)
50 nM intercalating fluorescent dye-labeled nucleic acid probe (SEQ ID NO: 42)
0.16 µM cleavage oligonucleotide (SEQ ID NO: 50): The 3-terminal -OH of this oligonucleotide was modified with an amino group.
6 U/30 µl ribonuclease inhibitor (Takara Bio Inc.) 11.7% DMSO

Reaction mixture composition (B): Concentrations are final concentrations (in 30 µl) after addition of enzyme solution.
60 mM Tris-HCl (pH 8.6)
18 mM magnesium chloride
100 mM potassium chloride
1 mM DTT
0.25 mM dATP, dCTP, dGTP, dTTP each
3 mM ATP, CTP, UTP, GTP each
3.6 mM ITP
0.2 µM first primer (SEQ ID NO: 18): The first primer included the T7 polymerase-promoter sequence (SEQ ID NO: 65) added at the 5' end of the indicated nucleotide sequence.
0.2 µM second primer (SEQ ID NO: 33)
50 nM intercalating fluorescent dye-labeled nucleic acid probe (SEQ ID NO: 42)
0.16 µM cleavage oligonucleotide (SEQ ID NO: 54): The 3-terminal -OH of this oligonucleotide was modified with an amino group.
6 U/30 µl ribonuclease inhibitor (Takara Bio, Inc.) 13% DMSO

(3) These reaction mixtures (A, B) were kept at 43°C for 5 minutes, and then 5 µl of enzyme solution with the following composition, kept at 43°C for 2 minutes, was added.
Enzyme solution composition: Final concentrations at time of reaction (in 30 µl)
2% sorbitol
6.4 U/30 µl AMV reverse transcriptase (Life Science Co., Ltd.)
142 U/30 µl T7 RNA polymerase (Invitrogen Corp.) 3.6 µg/30 µl bovine serum albumin

(4) Next, a fluorescent spectrophotometer equipped with a heat controlling function and allowing direct measurement of the PCR tube was used for reaction at 43°C while periodically measuring the fluorescent intensity of the reaction mixture (excitation wavelength: 470 nm, fluorescent wavelength: 520 nm).

The time-related change in the fluorescent intensity ratio of the reaction mixture (fluorescent intensity value at prescribed time ÷ background fluorescent intensity value) is shown in Fig. 2, where the point of enzyme addition is defined as 0 minutes. Based on the results in Figs. 2A and B, a fluorescence profile dependent on the initial concentration of Reg IV RNA was obtained, and 50 copies/test of Reg IV RNA could be detected in approximately 10 minutes. This indicates that Reg IV RNA can be assayed at high sensitivity and high speed by the method of the invention.

### Example 4

Reg IV RNA was assayed by the method of the invention using different combinations of first primer, second primer, intercalating fluorescent dye-labeled nucleic acid probe and cleavage oligonucleotide.

(1) The aforementioned Reg IV RNA (including base numbers 1-1275) was diluted to 10⁴, 10² or 50 copies/5 µl using an RNA diluent (10 mM Tris-HCl (pH 8.0), 1 mM EDTA, 0.25 U/µl ribonuclease inhibitor, 5.0 mM DTT), for use as RNA samples.

(2) After dispensing 20 µl of reaction mixture with the following composition into a 0.5 ml-volume PCR tube (GeneAmp Thin-Walled Reaction Tubes, Perkin-Elmer), 5 µl of RNA sample was added.

Reaction mixture composition: Concentrations are final concentrations (in 30 µl) after addition of enzyme solution.
60 mM Tris-HC1 (pH 8.6)
18 mM magnesium chloride
100 mM potassium chloride
1 mM DTT
0.25 mM dATP, dCTP, dGTP, dTTP each
3 mM ATP, CTP, UTP, GTP each
3.6 mM ITP
1 µM first primer (SEQ ID NO. as listed in Tables 1 to 3): The first primer included the T7 polymerase-promoter sequence (SEQ ID NO: 65) added at the 5' end of the indicated nucleotide sequence.
1 µM second primer (SEQ ID NO. as listed in Tables 1 to 3)
20 nM intercalating fluorescent dye-labeled nucleic acid probe (SEQ ID NO. as listed in Tables 1 to 3): Nucleic acid probe prepared in Example 2.
0.16 µM cleavage oligonucleotide (SEQ ID NO. as listed in Tables 1 to 3): The 3-terminal -OH of the oligonucleotide was modified with an amino group.
6 U/30 µl ribonuclease inhibitor (Takara Bio Inc.) 13% DMSO

(3) The reaction mixture was kept at 43°C for 5 minutes, and then 5 µl of enzyme solution with the following composition, kept at 43°C for 2 minutes, was added.
Enzyme solution composition: Final concentrations at time of reaction (in 30 µl)
2% sorbitol
6.4 U/30 µl AMV reverse transcriptase (Life Science Co., Ltd.)
142 U/30 µl T7 RNA polymerase (Invitrogen Corp.)
3.6 µg/30 µl bovine serum albumin

(4) Next, a fluorescent spectrophotometer equipped with a heat controlling function and allowing direct measurement of the PCR tube was used for reaction at 43°C while periodically measuring the fluorescent intensity of the reaction mixture (excitation wavelength: 470 nm, fluorescent wavelength: 520 nm). Tables 1 to 3 shows the results, where (+) indicates that the fluorescent intensity ratio of the reaction mixture exceeded 1.2, and the time at that point is listed as the detection time, with the point of enzyme addition defined as 0 minutes.

When using the combinations of first primer, second primer, intercalating fluorescent dye-labeled nucleic acid probe and cleavage oligonucleotide listed in Table 1, 10⁴, 10² and 50 copies/test of Reg IV RNA were detected within 30 minutes. Specifically, Reg IV RNA could be rapidly detected when using a combination comprising a sequence selected from among SEQ ID NOS: 10 and 12-23 as the first primer, a sequence selected from among SEQ ID NOS: 11 and 30-33 as the second primer, a sequence selected from among SEQ ID NOS: 42, 43 as the intercalating fluorescent dye-labeled nucleic acid probe and a sequence selected from among SEQ ID NOS: 49-58 as the cleavage oligonucleotide, a combination comprising the sequence listed as SEQ ID NO: 3 as the first primer, a sequence selected from among SEQ ID NOS: 34, 35 as the second primer, a sequence selected from among SEQ ID NOS: 44, 45 as the intercalating fluorescent dye-labeled nucleic acid probe and a sequence selected from SEQ ID NO: 59 as the cleavage oligonucleotide, or a combination comprising a sequence selected from among SEQ ID NOS: 24-29 as the first primer, a sequence selected from among SEQ ID NOS: 36-41 as the second primer, a sequence selected from among SEQ ID NOS: 46-48 as the intercalating fluorescent dye-labeled nucleic acid probe and a sequence selected from among SEQ ID NOS: 60-64 as the cleavage oligonucleotide. Here, SEQ ID NOS: 10 and 12-23 are each partial sequences of SEQ ID NO: 1, SEQ ID NOS: 11 and 30-33 are each partial sequences of SEQ ID NO: 2, SEQ ID NOS: 42, 43 are each partial sequences of SEQ ID NO: 7, SEQ ID NOS: 34, 35 are each partial sequences of SEQ ID NO: 4, SEQ ID NOS: 44, 45 are each partial sequences of SEQ ID NO: 8, SEQ ID NOS: 24-29 are each partial sequences of SEQ ID NO: 5, SEQ ID NOS: 36-41 are each partial sequences of SEQ ID NO: 5, and SEQ ID NOS: 46-48 are each partial sequences of SEQ ID NO: 9. This indicated that the RNA amplification and assay method using the first primers, second primers and intercalating fluorescent dye-labeled nucleic acid probes of the invention allow rapid detection of Reg IV RNA.

Tables 1 to 3: Results of assays using different oligonucleotide combinations

**Table 1**

| Oligonucleotide combination | | | | Assay results | |
|---|---|---|---|---|---|
| First primer (SEQ ID NO) | Second primer (SEQ ID NO) | Intercalating fluorescent dye-labeled fluorescent probe (SEQ ID NO) | Cleavage oligonucleotide (SEQ ID NO) | 10' copy detection time | Intensity ratio |
| 3 | 34 | 44 | 59 | 20.39 | (+) |
| 3 | 34 | 45 | 59 | 20.48 | (+) |
| 3 | 35 | 44 | 59 | 12.7 | (+) |
| 3 | 35 | 45 | 59 | 15.95 | (+) |
| 22 | 11 | 42 | 57 | 8.55 | (+) |
| 22 | 32 | 43 | 57 | 14.96 | (+) |
| 22 | 32 | 42 | 57 | 9.68 | (+) |
| 23 | 11 | 42 | 58 | 11.39 | (+) |
| 23 | 31 | 42 | 58 | 10.58 | (+) |
| 24 | 37 | 48 | 60 | 21.65 | (+) |
| 24 | 39 | 48 | 60 | 18.67 | (+) |
| 25 | 36 | 48 | 61 | 12.05 | (+) |
| 25 | 38 | 48 | 61 | 13.93 | (+) |
| 25 | 39 | 48 | 61 | 13.86 | (+) |
| 25 | 40 | 48 | 61 | 12.9 | (+) |
| 25 | 41 | 48 | 61 | 18.17 | (+) |
| 26 | 36 | 48 | 61 | 15.25 | (+) |
| 26 | 38 | 48 | 61 | 24.44 | (+) |
| 26 | 39 | 48 | 61 | 16.66 | (+) |
| 26 | 40 | 48 | 61 | 18.07 | (+) |
| 26 | 41 | 48 | 61 | 27.22 | (+) |
| 27 | 36 | 46 | 62 | 18.4 | (+) |
| 27 | 36 | 47 | 62 | 17.08 | (+) |
| 27 | 37 | 46 | 62 | 20.92 | (+) |
| 27 | 37 | 47 | 62 | 19.17 | (+) |
| 27 | 37 | 48 | 62 | 13.62 | (+) |
| 27 | 38 | 48 | 62 | 15.33 | (+) |
| 27 | 39 | 46 | 62 | 18.53 | (+) |
| 27 | 39 | 47 | 62 | 17.18 | (+) |
| 27 | 39 | 48 | 62 | 12.88 | (+) |
| 27 | 40 | 48 | 62 | 12.29 | (+) |
| 27 | 41 | 48 | 62 | 15.52 | (+) |
| 28 | 36 | 48 | 63 | 13.26 | (+) |
| 28 | 39 | 48 | 63 | 14.58 | (+) |
| 28 | 40 | 48 | 63 | 14.74 | (+) |
| 28 | 41 | 48 | 63 | 27.69 | (+) |
| 29 | 36 | 46 | 64 | 25.08 | (+) |
| 29 | 36 | 47 | 64 | 19.31 | (+) |
| 29 | 36 | 48 | 64 | 15.64 | (+) |
| 29 | 37 | 46 | 64 | 25.03 | (+) |
| 29 | 37 | 47 | 64 | 23.01 | (+) |
| 29 | 39 | 46 | 64 | 29.47 | (+) |
| 29 | 39 | 47 | 64 | 22.92 | (+) |
| 29 | 39 | 48 | 64 | 16.63 | (+) |

**Table 2**

| Oligonucleotide combination | | | | Assay results | |
|---|---|---|---|---|---|
| First primer (SEQ ID NO) | Second primer (SEQ ID NO) | Intercalating fluorescent dye-labeled fluorescent probe (SEQ ID NO) | Cleavage oligonucleotide (SEQ ID NO) | 10² copy detection time | Intensity ratio |
| 13 | 11 | 42 | 50 | 13.23 | (+) |
| 14 | 11 | 42 | 51 | 13.12 | (+) |
| 14 | 31 | 42 | 51 | 11.78 | (+) |
| 14 | 33 | 42 | 51 | 23.57 | (+) |
| 15 | 11 | 42 | 52 | 11.31 | (+) |
| 15 | 31 | 42 | 52 | 11.69 | (+) |
| 16 | 32 | 42 | 53 | 10.56 | (+) |
| 16 | 32 | 43 | 53 | 18.98 | (+) |
| 17 | 11 | 42 | 54 | 10.53 | (+) |
| 17 | 31 | 42 | 54 | 11.32 | (+) |
| 17 | 32 | 42 | 54 | 12.41 | (+) |
| 18 | 11 | 42 | 54 | 9.8 | (+) |
| 18 | 30 | 42 | 54 | 12.98 | (+) |
| 18 | 31 | 42 | 54 | 8.76 | (+) |
| 18 | 32 | 42 | 54 | 11.75 | (+) |
| 18 | 33 | 42 | 54 | 8.59 | (+) |
| 19 | 11 | 42 | 55 | 17.2 | (+) |
| 19 | 31 | 42 | 55 | 9.07 | (+) |
| 19 | 32 | 42 | 55 | 12.87 | (+) |
| 19 | 33 | 42 | 55 | 11.43 | (+) |
| 20 | 11 | 42 | 55 | 9.78 | (+) |
| 20 | 31 | 42 | 55 | 12.27 | (+) |
| 20 | 32 | 42 | 55 | 11.67 | (+) |
| 20 | 33 | 42 | 55 | 11.68 | (+) |
| 21 | 11 | 42 | 56 | 29.99 | (+) |
| 21 | 31 | 42 | 56 | 27.86 | (+) |
| 21 | 32 | 42 | 56 | 19.91 | (+) |
| 21 | 33 | 42 | 56 | 12.59 | (+) |
| 22 | 31 | 42 | 57 | 14.3 | (+) |
| 22 | 32 | 42 | 57 | 16.58 | (+) |
| 22 | 33 | 42 | 57 | 12.24 | (+) |
| 23 | 32 | 42 | 58 | 14.59 | (+) |
| 23 | 33 | 42 | 58 | 14.11 | (+) |
| 27 | 36 | 48 | 62 | 13.84 | (+) |
| 27 | 39 | 48 | 62 | 15.1 | (+) |
| 29 | 37 | 48 | 64 | 19.85 | (+) |

**Table 3**

| Oligonucleotide combination | | | | Assay results | |
|---|---|---|---|---|---|
| First primer (SEQ ID NO) | Second primer (SEQ ID NO) | Intercalating fluorescent dye-labeled fluorescent probe (SEQ ID NO) | Cleavage oligonucleotide (SEQ ID NO) | 50 copy detection time | Intensity ratio |
| 10 | 11 | 42 | 50 | 8.79 | (+) |
| 10 | 31 | 42 | 50 | 10.39 | (+) |
| 10 | 32 | 42 | 50 | 12.09 | (+) |
| 12 | 11 | 42 | 49 | 8.7 | (+) |
| 12 | 30 | 42 | 49 | 18.39 | (+) |
| 19 | 30 | 42 | 55 | 12.64 | (+) |

| | | | | | |
|---|---|---|---|---|---|
| The oligonucleotide combinations listed above were used for RNA amplification/fluorescent measurement with 10⁴, 10² and 50 copies/test REG IV RNA as samples. Samples with fluorescent intensity ratios >1.2 are indicated as (+), and the times at those points were recorded as the detection times. | | | | | |

The oligonucleotide combinations listed in the tables were used for RNA amplification and fluorescence measurement with 50, 10² and 10⁴ copies/test of Reg IV RNA as samples. Samples with a fluorescent intensity ratio exceeding 1.2 were indicated as (+), and the time at that point was recorded as the detection time.

### Example 5

The method of the invention was used for assay of Reg IV RNA.

(1) The aforementioned Reg IV RNA (including base numbers 7-1242) was diluted to 10², 10³, 10⁴, 10⁵ and 10⁶ copies/5 µl using an RNA diluent (10 mM Tris-HCl (pH 8.0), 1 mM EDTA, 0.25 U/µl ribonuclease inhibitor, 5.0 mM DTT), for use as calibration curve standard RNA. The diluent was used as a negative standard (NEG). Samples A (10² copies/5 µl), B (10³ copies-/5 µl), C (10⁴ copies/5 µl), D (10⁵ copies/5 µl) and E (10⁶ copies/5 µl) were prepared in the same manner.

(2) After dispensing 20 µl of reaction mixture with the following composition into a 0.5 ml-volume PCR tube (GeneAmp Thin-Walled Reaction Tubes, Perkin-Elmer), the standard RNA and 5 µl of sample were added.
Reaction mixture composition (A): Concentrations are final concentrations (in 30 µl) after addition of enzyme solution.
60 mM Tris-HCl (pH 8.6)
18 mM magnesium chloride
100 mM potassium chloride
1 mM DTT
0.25 mM dATP, dCTP, dGTP, dTTP each
3 mM ATP, CTP, UTP, GTP each
3.6 mM ITP
0.2 µM first primer (SEQ ID NO: 10): The first primer included the T7 polymerase-promoter sequence (SEQ ID NO: 65) added at the 5' end of the indicated nucleotide sequence.
0.2 µM second primer (SEQ ID NO: 11)
50 nM intercalating fluorescent dye-labeled nucleic acid probe (SEQ ID NO: 42): Nucleic acid probe of Example 2 with oxazole yellow bonded via a linker to the phosphate diester.
0.16 µM cleavage oligonucleotide (SEQ ID NO: 50): The 3-terminal -OH of this oligonucleotide was modified with an amino group.
6 U/30 µl ribonuclease inhibitor (Takara Bio Inc.) 11.7% DMSO
Reaction mixture composition (B): Concentrations are final concentrations (in 30 µl) after addition of enzyme solution.
60 mM Tris-HCl (pH 8.6)
18 mM magnesium chloride
100 mM potassium chloride
1 mM DTT
0.25 mM dATP, dCTP, dGTP, dTTP each
3 mM ATP, CTP, UTP, GTP each
3.6 mM ITP
0.2 µM first primer (SEQ ID NO: 18): The first primer included the T7 polymerase-promoter sequence (SEQ ID NO: 65) added at the 5' end of the indicated nucleotide sequence.
0.2 µM second primer (SEQ ID NO: 33)
50 nM intercalating fluorescent dye-labeled nucleic acid probe (SEQ ID NO: 42)
0.16 µM cleavage oligonucleotide (SEQ ID NO: 54): The 3-terminal -OH of this oligonucleotide was modified with an amino group.
6 U/30 µl ribonuclease inhibitor (Takara Bio Inc.) 13% DMSO

(3) These reaction mixtures (A, B) were kept at 43°C for 5 minutes, and then 5 µl of enzyme solution with the following composition, kept at 43°C for 2 minutes, was added.
Enzyme solution composition: Final concentrations at time of reaction (30 µl)
2% sorbitol
6.4 U/30 µl AMV reverse transcriptase (Life Science Co., Ltd.)
142 U/30 µl T7 RNA polymerase (Invitrogen Corp.)
3.6 µg/30 µl bovine serum albumin

(4) Next, a fluorescent spectrophotometer equipped with a heat controlling function and allowing direct measurement of the PCR tube was used for reaction at 43°C while periodically measuring the fluorescent intensity of the reaction mixture (excitation wavelength: 470 nm, fluorescent wavelength: 520 nm). The time-related change in the fluorescent intensity ratio of the reaction mixture (fluorescent intensity value at prescribed time ÷ background fluorescent intensity value) is shown in Figs. 3A and B, where the point of enzyme addition is defined as 0 minutes.

With the detection time defined as the time at which the fluorescent intensity ratio reached 1.2 based on the results in Figs. 3A and B, a calibration curve was drawn from the detection time and logarithm of initial number of copies, as shown in Figs. 4A and B. The numbers of copies for samples A, B, C, D and E were determined from the calibration curve, yielding the results shown in Table 4.

In the results shown in Figs. 4A and B, 10² copies/test were detected at approximately 9 minutes, and a satisfactory correlation was obtained between detection time and log of initial number of copies. In the results shown in Tables 4A and B, the assay results for samples A, B, C, D and E are values corresponding to the amounts of Reg IV RNA added. Thus, it was demonstrated that the method of the invention can assay Reg IV RNA in a rapid, highly sensitive and specific manner.

### Table 4

### REG IV RNA quantitation

A)

| Sample | Assay results | |
|---|---|---|
| | Detection time (min) | Number of copies/test |
| A | 8.87 | 2.5 x 10² |
| B | 8.23 | 1.3 x 10³ |
| C | 7.27 | 1.4 x 10⁴ |
| D | 6.44 | 1.2 x 10⁵ |
| E | 5.60 | 9.8 x 10⁵ |

| | | |
|---|---|---|
| Results for detection times obtained from the fluorescence profiles resulting from measurement of samples A (10² copies/5 µl), B (10³ copies/5 µl), C (10⁴ copies/5 µl), D (10⁵ copies/5 µl) and E (10⁶ copies/5 µl), and numbers of copies calculated from calibration curve of Fig. 4A. | | |

B)

| Sample | Assay results | |
|---|---|---|
| | Detection time (min) | Number of copies/test |
| A | 8.88 | 1.5 x 10² |
| B | 7.88 | 1.6 x 10³ |
| C | 6.89 | 1.6 x 10⁴ |
| D | 6.04 | 1.2 x 10⁵ |
| E | 5.23 | 8.5 x 10⁵ |

| | | |
|---|---|---|
| Results for detection times obtained from the fluorescence profiles resulting from measurement of samples A (10² copies/5 µl), B (10³ copies/5 µl), C (10⁴ copies/5 µl), D (10⁵ copies/5 µl) and E (10⁶ copies/5 µl), and numbers of copies calculated from calibration curve of Fig. 4B. | | |

Results of calculating detection time from fluorescence profile based on measurement results for samples A (10² copies/5 µl), B (10³ copies/5 µl), C (10⁴ copies/5 µl), D (10⁵ copies/5 µl) and E (10⁶ copies/5 µl), and number of copies from the calibration curve of Fig. 4. Table 4A shows the results using reaction mixture composition (A), and Table 4B shows the results using reaction mixture composition (B).

### Industrial Applicability

The present invention can be applied as a marker for gastric cancer, colon cancer and other cancers.

## Claims

1. A method for assaying Regenerating islet-derived family, member 4 (regenerating gene type 4) mRNA (Reg IV mRNA) present in a sample, the method being **characterized by** comprising
(1) a step of using a first primer homologous to at least a portion downstream from the 5' end of a specified nucleotide sequence of said RNA and a second primer complementary to at least a portion upstream from the 3' end of said specified nucleotide sequence, to produce double-stranded DNA containing a promoter sequence and said specified nucleotide sequence downstream from said promoter sequence, where at least one of said first and second primers has said promoter sequence at the 5' end,
(2) a step of using said double-stranded DNA as template to produce an RNA transcript,
(3) a step of using said RNA transcript in turn as template for DNA synthesis, to amplify said RNA transcript in a chain-reaction, and
(4) a step of assaying the amount of said RNA transcript.

2. A method for assaying Reg IV mRNA according to claim 1, **characterized by** employing a combination of said first primer which includes at least 15 contiguous bases of the sequence listed as SEQ ID NO: 1 and said second primer which includes at least 15 bases of the sequence listed as SEQ ID NO: 2, a combination of said first primer which includes at least 15 contiguous bases of the sequence listed as SEQ ID NO: 3 and said second primer which includes at least 15 bases of the sequence listed as SEQ ID NO: 4, or a combination of said first primer which includes at least 15 contiguous bases of the sequence listed as SEQ ID NO: 5 and said second primer which includes at least 15 bases of the sequence listed as SEQ ID NO: 6.

3. A method for assaying Reg IV mRNA according to claim 1, **characterized in that** the assay of the amount of said RNA transcript is accomplished by measuring the change in the fluorescent property of a nucleic acid probe that is labeled with an intercalating dye and is designed so that when it forms a complementary double strand with the target nucleic acid, the intercalating fluorescent dye portion undergoes a change in fluorescent property by intercalating into said complementary double strand.

4. A method for assaying Reg IV mRNA according to claim 3, **characterized by** employing a combination of sequences of said first primer which includes at least 15 contiguous bases of the sequence listed as SEQ ID NO: 1, said second primer which includes at least 15 bases of the sequence listed as SEQ ID NO: 2 and the intercalating fluorescent dye-labeled nucleic acid probe which includes at least 15 bases of the sequence listed as SEQ ID NO: 7 or of the sequence complementary to said sequence, a combination of sequences of said first primer which includes at least 15 contiguous bases of the sequence listed as SEQ ID NO: 3, said second primer which includes at least 15 bases of the sequence listed as SEQ ID NO: 4 and the intercalating fluorescent dye-labeled nucleic acid probe which includes at least 15 bases of the sequence listed as SEQ ID NO: 8 or the sequence complementary to said sequence, or a combination of sequences of said first primer which includes at least 15 contiguous bases of the sequence listed as SEQ ID NO: 5, said second primer which includes at least 15 bases of the sequence listed as SEQ ID NO: 6 and the intercalating fluorescent dye-labeled nucleic acid probe which includes at least 15 bases of the sequence listed as SEQ ID NO: 9 or the sequence complementary to said sequence.

5. An assay reagent for Reg IV mRNA, **characterized in that** its essential constituent elements are oligonucleotides comprising a combination of sequences of a first primer which includes at least 15 contiguous bases of the sequence listed as SEQ ID NO: 1 and a second primer which includes at least 15 contiguous bases of the sequence listed as SEQ ID NO: 2, where at least one of said first and second primers has a promoter sequence at the 5' end, a combination of sequences of a first primer which includes at least 15 contiguous bases of the sequence listed as SEQ ID NO: 3 and a second primer which includes at least 15 contiguous bases of the sequence listed as SEQ ID NO: 4, where at least one of said first and second primers has a promoter sequence at the 5' end, or a combination of sequences of a first primer which includes at least 15 contiguous bases of the sequence listed as SEQ ID NO: 5 and a second primer which includes at least 15 contiguous bases of the sequence listed as SEQ ID NO: 6, where at least one of said first and second primers has a promoter sequence at the 5' end.

6. A method for assaying Reg IV mRNA, **characterized in that** its essential constituent elements are oligonucleotides comprising a combination of sequences of said first primer which includes at least 15 contiguous bases of the sequence listed as SEQ ID NO: 1, said second primer which includes at least 15 bases of the sequence listed as SEQ ID NO: 2 and the intercalating fluorescent dye-labeled nucleic acid probe which includes at least 15 bases of the sequence listed as SEQ ID NO: 7 or of the sequence complementary to said sequence, where at least one of said first and second primers has a promoter sequence at the 5' end, a combination of sequences of said first primer which includes at least 15 contiguous bases of the sequence listed as SEQ ID NO: 3, said second primer which includes at least 15 bases of the sequence listed as SEQ ID NO: 4 and the intercalating fluorescent dye-labeled nucleic acid probe which includes at least 15 bases of the sequence listed as SEQ ID NO: 8 or of the sequence complementary to said sequence, where at least one of said first and second primers has a promoter sequence at the 5' end, or a combination of sequences of said first primer which includes at least 15 contiguous bases of the sequence listed as SEQ ID NO: 5, said second primer which includes at least 15 bases of the sequence listed as SEQ ID NO: 6 and the intercalating fluorescent dye-labeled nucleic acid probe which includes at least 15 bases of the sequence listed as SEQ ID NO: 9 or of the sequence complementary to said sequence, where at least one of said first and second primers has a promoter sequence at the 5' end.

7. An oligonucleotide for amplification or detection of Reg IV mRNA, comprising at least 15 contiguous bases of any of the nucleotide sequences listed as SEQ ID NOS: 1 to 9 or the sequences complementary to those sequences.


SEQUENCE LISTING

<110>  TOSOH CORPORATION

<120>  Method for Determining REG IV mRNA

<130>  PA211

<150>  JP 2005-314343
<151>  2005-10-28

<160>  65

<170>  PatentIn version 3.3

<210>  1
<211>  84
<212>  DNA
<213>  Artificial Sequence

<220>
<223>  First Primer

<400>  1
ttatcgcttt gtgactaaag taaagattat taattcctga ggcaagaaga tataaaagct      60

ccagaaacgt tgactgggac cact                                              84


<210>  2
<211>  49
<212>  DNA
<213>  Artificial Sequence

<220>

<223>  Second Primer

<400>  2
gggttctcct tgatctgcaa atctgtttgg caaccaagac tctaagggc                49

<210>  3
<211>  20
<212>  DNA
<213>  Artificial Sequence

<220>
<223>  First Primer

<400>  3
cttggttgcc aaacagattt                                                20

<210>  4
<211>  40
<212>  DNA
<213>  Artificial Sequence

<220>
<223>  Second Primer

<400>  4
atgcttctgg aagccatctt cctcctaccc tgaggatgta                          40

<210>  5
<211>  48
<212>  DNA
<213>  Artificial Sequence

<220>
<223>  First Primer

<400>  5
acagaagagg cagcagtggc agtggattga tggggccatg tatctgta                48

<210>  6
<211>  75
<212>  DNA
<213>  Artificial Sequence

<220>
<223>  Second Primer

<400>  6
cttgattctt gctctatggt cggtacttgc acaggaagtg ttggcgcttg ttgcattcgt      60

tgctgctcca agtta                                                      75

<210>  7
<211>  31
<212>  DNA
<213>  Artificial Sequence

<220>
<223>  Intercalator Fluorescent Dye-Labeled Nucleic Acid Probe

<400>  7
taagggcccc tgccttcttc agtgtctcca g                                    31

<210>  8
<211>  43

<212>  DNA
<213>  Artificial Sequence

<220>
<223>  Intercalator Fluorescent Dye-Labeled Nucleic Acid Probe

<400>  8
ggatctcaga gaccttacta gcgcttcttt gaaactcctg ggt                43

<210>  9
<211>  60
<212>  DNA
<213>  Artificial Sequence

<220>
<223>  Intercalator Fluorescent Dye-Labeled Nucleic Acid Probe

<400>  9
tgttattgga gctcatctca gcacagtgct tgttcccacc catggacttg ccagaccagg      60

<210>  10
<211>  28
<212>  DNA
<213>  Artificial Sequence

<220>
<223>  First Primer

<400>  10
tatcgctttg tgactaaagt aaagatta                                 28

<210>  11

<211>  21
<212>  DNA
<213>  Artificial Sequence

<220>
<223>  Second Primer

<400>  11
ggcaaccaag actctaaggg c                                                21


<210>  12
<211>  28
<212>  DNA
<213>  Artificial Sequence

<220>
<223>  First Primer

<400>  12
ttatcgcttt gtgactaaag taaagatt                                         28


<210>  13
<211>  25
<212>  DNA
<213>  Artificial Sequence

<220>
<223>  First Primer

<400>  13
cgctttgtga ctaaagtaaa gatta                                            25

<210>  14
<211>  25
<212>  DNA
<213>  Artificial Sequence

<220>
<223>  First Primer

<400>  14
taaagattat taattcctga ggcaa                                          25


<210>  15
<211>  25
<212>  DNA
<213>  Artificial Sequence

<220>
<223>  First Primer

<400>  15
attcctgagg caagaagata taaaa                                          25


<210>  16
<211>  25
<212>  DNA
<213>  Artificial Sequence

<220>
<223>  First Primer

<400>  16
ctgaggcaag aagatataaa agctc                                          25

<210>  17
<211>  24
<212>  DNA
<213>  Artificial Sequence

<220>
<223>  First Primer

<400>  17
caagaagata taaaagctcc agaa                                              24

<210>  18
<211>  24
<212>  DNA
<213>  Artificial Sequence

<220>
<223>  First Primer

<400>  18
aagaagatat aaaagctcca gaaa                                              24

<210>  19
<211>  24
<212>  DNA
<213>  Artificial Sequence

<220>
<223>  First Primer

<400>  19
agaagatata aaagctccag aaac                                              24

<210>  20
<211>  20
<212>  DNA
<213>  Artificial Sequence

<220>
<223>  First Primer

<400>  25
gatataaaag ctccagaaac gttgac                                          25

<210>  21
<211>  23
<212>  DNA
<213>  Artificial Sequence

<220>
<223>  First Primer

<400>  21
agctccagaa acgttgactg gga                                             23

<210>  22
<211>  20
<212>  DNA
<213>  Artificial Sequence

<220>
<223>  First Primer

<400>  22

tccagaaacg ttgactggga 20

<210> 23
<211> 22
<212> DNA
<213> Artificial Sequence

<220>
<223> First Primer

<400> 23
agaaacgttg actgggacca ct 22

<210> 24
<211> 19
<212> DNA
<213> Artificial Sequence

<220>
<223> First Primer

<400> 24
acagaagagg cagcagtgg 19

<210> 25
<211> 20
<212> DNA
<213> Artificial Sequence

<220>
<223> First Primer

<400>  25
ggcagcagtg gcagtggatt                                                    20

<210>  26
<211>  20
<212>  DNA
<213>  Artificial Sequence

<220>
<223>  First Primer

<400>  26
cagtggcagt ggattgatgg                                                    20

<210>  27
<211>  19
<212>  DNA
<213>  Artificial Sequence

<220>
<223>  First Primer

<400>  27
agtggcagtg gattgatgg                                                     19

<210>  28
<211>  19
<212>  DNA
<213>  Artificial Sequence

<220>
<223>  First Primer

<400>  28
tggattgatg gggccatgt                                                    19

<210>  29
<211>  20
<212>  DNA
<213>  Artificial Sequence

<220>
<223>  First Primer

<400>  29
gatggggcca tgtatctgta                                                   20

<210>  30
<211>  23
<212>  DNA
<213>  Artificial Sequence

<220>
<223>  Second Primer

<400>  30
ctgtttggca accaagactc taa                                               23

<210>  31
<211>  20
<212>  DNA
<213>  Artificial Sequence

<220>

<223>  Second Primer

<400>  31
ctgtttggca accaagactc                                                    20


<210>  32
<211>  25
<212>  DNA
<213>  Artificial Sequence

<220>
<223>  Second Primer

<400>  32
gttctccttg atctgcaaat ctgtt                                              25


<210>  33
<211>  27
<212>  DNA
<213>  Artificial Sequence

<220>
<223>  Second Primer

<400>  33
gggttctcct tgatctgcaa atctgtt                                            27


<210>  34
<211>  20
<212>  DNA
<213>  Artificial Sequence

<220>
<223>  Second Primer

<400>  34
cctcctaccc tgaggatgta                                                    20


<210>  35
<211>  20
<212>  DNA
<213>  Artificial Sequence

<220>
<223>  Second Primer

<400>  35
atgcttctgg aagccatctt                                                    20


<210>  36
<211>  20
<212>  DNA
<213>  Artificial Sequence

<220>
<223>  Second Primer

<400>  36
ttcgttgctg ctccaagtta                                                    20


<210>  37
<211>  19
<212>  DNA
<213>  Artificial Sequence

<220>
<223>  Second Primer

<400>  37
aagtgttggc gcttgttgc                                                    19

<210>  38
<211>  23
<212>  DNA
<213>  Artificial Sequence

<220>
<223>  Second Primer

<400>  38
gctctatggt cggtacttgc aca                                               23

<210>  39
<211>  21
<212>  DNA
<213>  Artificial Sequence

<220>
<223>  Second Primer

<400>  39
cttgctctat ggtcggtact t                                                 21

<210>  40
<211>  26
<212>  DNA

<213>  Artificial Sequence

<220>
<223>  Second Primer

<400>  40
tgattcttgc tctatggtcg gtactt                                          26

<210>  41
<211>  23
<212>  DNA
<213>  Artificial Sequence

<220>
<223>  Second Primer

<400>  41
cttgattctt gctctatggt cgg                                             23

<210>  42
<211>  20
<212>  DNA
<213>  Artificial Sequence

<220>
<223>  Intercalator Fluorescent Dye-Labeled Nucleic Acid Probe

<400>  42
gccttcttca gtgtctccag                                                 20

<210>  43
<211>  18

<212>  DNA
<213>  Artificial Sequence

<220>
<223>  Intercalator Fluorescent Dye-Labeled Nucleic Acid Probe

<400>  43
taagggcccc tgccttct                                                    18


<210>  44
<211>  21
<212>  DNA
<213>  Artificial Sequence

<220>
<223>  Intercalator Fluorescent Dye-Labeled Nucleic Acid Probe

<400>  44
gcttctttga aactcctggg t                                                21


<210>  45
<211>  23
<212>  DNA
<213>  Artificial Sequence

<220>
<223>  Intercalator Fluorescent Dye-Labeled Nucleic Acid Probe
<400>  45
ggatctcaga gaccttacta gcg                                              23


<210>  46
<211>  24

<212>  DNA
<213>  Artificial Sequence

<220>
<223>  Intercalator Fluorescent Dye-Labeled Nucleic Acid Probe

<400>  46
atggacttgc cagaccagg                                                    19


<210>  47
<211>  20
<212>  DNA
<213>  Artificial Sequence

<220>
<223>  Intercalator Fluorescent Dye-Labeled Nucleic Acid Probe
<400>  47
tctcagcaca gtgcttgttc                                                   20


<210>  48
<211>  22
<212>  DNA
<213>  Artificial Sequence

<220>
<223>  Intercalator Fluorescent Dye-Labeled Nucleic Acid Probe

<400>  48
tgttattgga gctcatctca gc                                                22


<210>  49
<211>  26

<212>  DNA
<213>  Artificial Sequence

<220>
<223>  Cleavage Oligonucleotide

<400>  49
gcgataatat tatttgagaa aaacaa                                          26

<210>  50
<211>  26
<212>  DNA
<213>  Artificial Sequence

<220>
<223>  Cleavage Oligonucleotide

<400>  50
aaagcgataa tattatttga gaaaaa                                          26

<210>  51
<211>  26
<212>  DNA
<213>  Artificial Sequence

<220>
<223>  Cleavage Oligonucleotide

<400>  51
tctttacttt agtcacaaag cgataa                                          26

<210>  52

<211>  26
<212>  DNA
<213>  Artificial Sequence

<220>
<223>  Cleavage Oligonucleotide

<400>  52
aggaattaat aatctttact ttagtc                                          26


<210>  53
<211>  25
<212>  DNA
<213>  Artificial Sequence

<220>
<223>  Cleavage Oligonucleotide

<400>  53
cctcaggaat taataatctt tactt                                           25


<210>  54
<211>  26
<212>  DNA
<213>  Artificial Sequence

<220>
<223>  Cleavage Oligonucleotide

<400>  54
cttcttgcct caggaattaa taatct                                          26

<210>  55
<211>  26
<212>  DNA
<213>  Artificial Sequence

<220>
<223>  Cleavage Oligonucleotide

<400>  55
tatatcttct tgcctcagga attaat                                          26


<210>  56
<211>  26
<212>  DNA
<213>  Artificial Sequence

<220>
<223>  Cleavage Oligonucleotide

<400>  56
ggagctttta tatcttcttg cctcag                                          26


<210>  57
<211>  25
<212>  DNA
<213>  Artificial Sequence

<220>
<223>  Cleavage Oligonucleotide

<400>  57
tctggagctt ttatatcttc ttgcc                                           25

<210> 58
<211> 26
<212> DNA
<213> Artificial Sequence

<220>
<223> Cleavage Oligonucleotide

<400> 58
gtttctggag cttttatatc ttcttg 26

<210> 59
<211> 25
<212> DNA
<213> Artificial Sequence

<220>
<223> Cleavage Oligonucleotide

<400> 59
gtttggcaac caagactcta agggc 25

<210> 60
<211> 25
<212> DNA
<213> Artificial Sequence

<220>
<223> Cleavage Oligonucleotide

<400> 60
ttctgtgggt cgtgcaggcc aatcc 25

<210> 61
<211> 26
<212> DNA
<213> Artificial Sequence

<220>
<223> Cleavage Oligonucleotide

<400> 61
gctgcctctt ctgtgggtcg tgcagg 26

<210> 62
<211> 25
<212> DNA
<213> Artificial Sequence

<220>
<223> Cleavage Oligonucleotide

<400> 62
gccactgctg cctcttctgt gggtc 25

<210> 63
<211> 26
<212> DNA
<213> Artificial Sequence

<220>
<223> Cleavage Oligonucleotide

<400> 63

aatccactgc cactgctgcc tcttct 26

<210> 64
<211> 25
<212> DNA
<213> Artificial Sequence

<220>
<223> Cleavage Oligonucleotide
<400> 64
cccatcaatc cactgccact gctgc 25

<210> 65
<211> 28
<212> DNA
<213> Artificial Sequence

<220>
<223> T7 Polymerase Promoter Sequence

<400> 65
aattctaata cgactcacta tagggaga 28